# EUROPÄISCHE PATENTSCHRIFT

(11) **EP 1 252 354 B1**
(45) Veröffentlichungstag und Bekanntmachung des Hinweises auf die Patenterteilung: **24.08.2005**
(21) Anmeldenummer: 01902316.7
(22) Anmeldetag: 13.01.2001
(51) Int. Cl.: C23C 2/02

(54) **VERFAHREN ZUM HERSTELLEN EINES MIT EINER ZINKBESCHICHTUNG VERSEHENEN STAHLBANDES**
METHOD FOR PRODUCING A STEEL STRIP WHICH IS PROVIDED WITH A ZINC COATING
PROCEDE DE FABRICATION D'UN RUBAN D'ACIER DOTE D'UNE COUVERTURE DE ZINC

(30) Priorität: 28.01.2000 DE 10003680
(43) Veröffentlichungstag der Anmeldung: 30.10.2002
(73) Patentinhaber: ThyssenKrupp Stahl AG, 47166 Duisburg (DE)
(72) Erfinder: MEURER, Manfred, 47495 Rheinberg (DE); WARNECKE, Wilhelm, 46499 Hamminkeln (DE); MAGER, Peter, 56581 Ehlscheid (DE)
(74) Vertreter: Simons, Johannes
(86) Internationale Anmeldenummer: PCT/EP2001/000367
(87) Internationale Veröffentlichungsnummer: WO 2001/055469

(56) Entgegenhaltungen:
- EP-A- 0 037 143
- EP-A- 0 337 402
- EP-A- 0 545 049
- DE-C- 315 712
- DE-C- 3 828 911
- US-A- 5 529 810
- US-A- 5 849 408
- PATENT ABSTRACTS OF JAPAN vol. 1999, no. 09, 30. Juli 1999 (1999-07-30) & JP 11 117052 A (TANAKA GALVANIZING CO LTD), 27. April 1999 (1999-04-27)
- PATENT ABSTRACTS OF JAPAN vol. 006, no. 106 (C-108), 16. Juni 1982 (1982-06-16) & JP 57 035672 A (NIPPON MINING CO LTD), 26. Februar 1982 (1982-02-26)
- PATENT ABSTRACTS OF JAPAN vol. 011, no. 003 (C-395), 7. Januar 1987 (1987-01-07) & JP 61 179861 A (SADAJI NAGABORI;OTHERS: 01), 12. August 1986 (1986-08-12)
- PATENT ABSTRACTS OF JAPAN vol. 013, no. 069 (C-569), 16. Februar 1989 (1989-02-16) & JP 63 262451 A (NIPPON TELEGR & TELEPH CORP ;OTHERS: 01), 28. Oktober 1988 (1988-10-28)
- DATABASE WPI Section Ch, Week 198445 Derwent Publications Ltd., London, GB; Class M13, AN 1984-278459 XP002166540 & JP 59 170249 A (NISSHIN STEEL CO LTD), 26. September 1984 (1984-09-26)
- DATABASE WPI Section Ch, Week 198445 Derwent Publications Ltd., London, GB; Class M13, AN 1984-279792 XP002166541 & JP 59 173253 A (SUMITOMO ELECTRIC IND CO), 1. Oktober 1984 (1984-10-01)

## Beschreibung

Die Erfindung betrifft ein Verfahren zum Herstellen eines mit einer Zinkbeschichtung versehenen Stahlbandes, bei dem das Band einem mindestens zweistufig erfolgenden Schmelztauchverzinken unterzogen wird. Darüber hinaus betrifft die Erfindung ein zinkbeschichtetes Stahlband.

Stahlbänder werden zur Verbesserung ihrer Korrosionsbeständigkeit für viele Anwendungsfälle mit einer Zinkbeschichtung versehen. Der Vorteil der Verwendung von Zink für die Beschichtung gegenüber anderen in Frage kommenden Beschichtungsmetallen besteht darin, daß Zink nach Art einer Opferanode einen aktiven und passiven Schutz des Stahlbands bietet. Eine weitere Verbesserung des Korrosionsschutzes konnte dadurch erreicht werden, daß eutektische Zn-Al-Legierungen mit Anteilen bis zu 5 % Aluminium auf das Stahlblech aufgetragen werden. Auf diese Weise konnten die hervorragenden Schutzeigenschaften des Aluminiums mit den ebenso günstigen Eigenschaften des Zinks vereinigt werden.

Ein grundsätzliches Problem bei der Beschichtung von Stahlbändern mit einer metallischen Korrosionsschutzschicht besteht darin, daß die Dicke der auf das Stahlband aufgetragenen Schutzschicht einerseits aufgrund des Fließverhaltens der metallischen Schmelze begrenzt ist. Andererseits besteht eine Ursache für diese Begrenzung darin, daß die sich auf der Oberfläche bildende Zinkschicht aufgrund ihrer Verbindung mit dem Eisen des Stahlbandes spröde ist. Diese Eigenschaft beeinträchtigt bei großen Beschichtungsdicken die Haftung der Beschichtung auf dem Stahlband, so daß es zu Abplatzungen kommt.

Eine Lösung dieses Problems besteht darin, das Stahlband einer zweistufigen Beschichtung zu ünterziehen. Ein solches sogenanntes "Double Dip"-Verfahren ist beispielsweise in dem Aufsatz "AGOZAL-ein neuartiger, verbesserter Korrosionsschutz für Bandstahl", veröffentlicht in der Zeitschrift "Stahl", Heft 2/1997, Seite 48-49, beschrieben. Bei diesem bekannten Verfahren wird das Stahlband in der ersten Stufe der Tauchschmelzverzinkung durch eine Zn-Schmelze geleitet, so daß sich auf der Oberfläche des Stahlbandes eine dicke Hartzinkschicht bildet, welche Eisen und Zink enthält. In der zweiten Stufe der Schmelztauchverzinkung wird das derart beschichtete Band durch eine ZnAl-Schmelze geleitet. Dabei soll die Eisen-Zinkschicht wie ein Flußmittel wirken, durch welches die Benetzbarkeit des Stahlbandes so verbessert wird. Auf diese Weise soll eine dicke ZnAl-Schicht auf das mit Zink vorbeschichtete Band aufgetragen werden können.

Das bekannte Verfahren geht weiter davon aus, daß sich im Zuge des Aufbringens der zweiten Zinkschicht die nach dem ersten Tauchverzinken auf dem Stahlband vorhandene spröde Hartzinkschicht durch Aufnahme von Teilen des beim zweiten Eintauchen gemeinsam mit dem Zink aufgetragenen Aluminiums in ein eutektisches Zn-Al-Gefüge wandelt, so daß nach der zweiten Stufe der Schmelztauchverzinkung eine dicke, aus Zink und Aluminium bestehende Beschichtung auf dem Band vorhanden ist. Diese soll ein einheitliches, homogenes Gefüge aufweisen und aufgrund der in der ersten Stufe des Schmelztauchens hergestellten innigen Verbindung zwischen dem Stahlband und der Zinkbeschichtung fest auf dem Stahlband haften.

Trotz der Vergrößerung der Dicke der Zink-Aluminium-Beschichtung, welche sich zumindest bei theoretischer Betrachtung durch das voranstehend erläuterte "Double-Dip"-Verfahren erzeugen läßt, hat sich in der Praxis herausgestellt, daß die Korrosionsbeständigkeit von entsprechend beschichteten Stahlbändern verbesserungswürdig ist.

Neben dem voranstehend erläuterten Stand der Technik ist aus der JP-A 11-117052 ein Verfahren zum zweistufig erfolgenden Stückverzinken von Stahlprodukten bekannt. Gemäß diesem Verfahren werden die jeweiligen Stahlprodukte stückweise einer Flux-Vorbehandlung mit einem Flußmittel für Zn-Al-Überzüge, das kein NH₄Cl enthält, unterzogen, bevor sie in ein erstes, einen Al-Gehalt von 0,01 bis 0,1 % aufweisendes Verzinkungsbad mit getaucht werden. In diesem ersten Verzinkungsschritt wird ein Überzug mit vermindertem Wachstum der Fe-Zn-Legierungsphase auf dem Stahlprodukt erzeugt. Anschließend werden die so grundbeschichteten Stahlprodukte mit Wasser abgekühlt, um anschließend in ein zweites Tauchbad getaucht zu werden. Dieses zweite Bad weist einen zwischen 3 % und 20 % liegenden Al-Gehalt und/oder einen Mg-Gehalt von 0,01 bis 1,0 % auf. Mit dem bekannten Verfahren soll ein Überzug erhalten werden, der keine Rauheiten oder Ablösungen aufweist und eine gute Korrosionsbeständigkeit bietet.

Die Aufgabe der Erfindung besteht darin, ausgehend von dem voranstehend erläuterten Stand der Technik ein Verfahren anzugeben, mit dem sich ein zinkbeschichtetes Stahlband herstellen läßt, welches einen verbesserten Korrosionsschutz bei gleichzeitig guten Umformeigenschaften gewährleistet. Das mit dem erfindungsgemäßen Verfahren erhaltene Stahlband soll ein hohes Umformvermögen und eine besonders gute Korrosionsbeständigkeit besitzen.

Diese Aufgabe wird durch ein Verfahren zum Herstellen eines mit einer Zinkbeschichtung versehenen Stahlbandes gelöst, bei dem das Stahlband kontinuierlich aufeinanderfolgend die folgenden Arbeitsschritte durchläuft und dabei einem mindestens zweistufig erfolgenden Schmelztauchverzinken unterzogen wird:
- Durchlaufglühen des Stahlbands vor dem Eintritt in das erste Tauchbad,
- Beschichten des Stahlbands in der ersten Stufe der Schmelztauchverzinkung mit einer Grundschicht, indem das Stahlband für eine erste Tauchzeit durch ein erstes Tauchbad aus einer einen niedrigen Aluminium-Gehalt aufweisenden Zinkschmelze geleitet wird,
- Abkühlen des mit der Grundschicht beschichteten Stahlbands und
- Aufbringen einer Deckschicht auf das mit der Grundschicht beschichtete Stahlband, indem in der zweiten Stufe der Schmelztauchbeschichtung das mit der Grundschicht beschichtete Stahlband für eine zweite Tauchzeit durch ein zweites Tauchbad geleitet wird, welches aus einer einen höheren Aluminium-Gehalt als die erste Zinkschmelze besitzenden zweiten Zinkschmelze gebildet ist.

Gemäß der Erfindung wird, anders als beim eingangs erläuterten sich auf die Tauchbeschichtung von Stahlbändern beziehenden Stand der Technik, gezielt eine mindestens zwei Schichten umfassende Beschichtung erzeugt. Dies wird dadurch erreicht, daß das Stahlband zunächst durch ein erstes Tauchbad geleitet wird, in welchem es mit einer ersten Zinkbeschichtung versehen wird. Anschließend wird das Band abgekühlt, so daß sich die beim ersten Eintauchen aufgebrachte Schicht verfestigt. Das derart mit einer festen Grundschicht versehene Band wird anschließend durch ein zweites Tauchverzinken mit der Deckschicht überzogen. Durch Diffusion bildet diese mit der Grundschicht die herzustellende Beschichtung des Stahlbands. Dabei kommt es, ebenfalls im Unterschied zum vorbekannten Stand der Technik, nicht zu einer Umwandlung der Grundschicht in ein der Deckschicht entsprechendes Gefüge, sondern das ursprüngliche Gefüge der Grundschicht nimmt durch Diffusion Aluminium auf und bleibt als eigenständige Schicht erhalten. Weitere Deckschichten können auf der ersten Deckschicht aufgebracht werden, indem das Stahlband durch weitere Tauchbäder geleitet wird. Dabei findet erforderlichenfalls zwischen den Tauchvorgängen jeweils eine Abkühlung des Bandes statt.

Aufgrund des schichtweisen Aufbaus der Beschichtung besitzt eine erfindungsgemäß erzeugte Beschichtung eine erhöhte Korrosionsbeständigkeit. So kann mit dem erfindungsgemäßen Verfahren einerseits eine gegenüber dem Stand der Technik verbesserte Dicke der Beschichtung von beispielsweise bis zu 80 µm erzeugt werden, was sich in der Praxis bisher nur durch die Verzinkung von Einzelstücken erreichen ließ. Die vergrößerte Dicke der Beschichtung führt zu einer Erhöhung der Dauer, über welche die Beschichtung auch in aggressiver Umgebung einen sicheren Schutz gewährleistet. Zum anderen können die einzelnen Schichten der Beschichtung so aufeinander abgestimmt werden, daß eine besonders feste Haftung der Beschichtung auf dem Stahlband sichergestellt ist. Gleichzeitig kann durch eine geeignete Abstimmung der Eigenschaften der Schichten die Möglichkeit einer Unterwanderung der Beschichtung im Fall einer Beschädigung eingeschränkt werden, welche andernfalls gerade bei großen Schichtdicken die Gefahr eines Abplatzens der Beschichtung mit sich bringt.

Die aus einer Aluminium-haltigen Zinkschmelze gebildete Deckschicht weist eine hohe Duktilität bei gleichzeitig optimalem Korrosionsschutz auf. Die Grundschicht ist ihrerseits ebenfalls duktil und weist gegenüber der Deckschicht einen zusätzlich erhöhten Korrosionswiderstand auf. Aufgrund der beiden Schichten gemeinsamen Duktilität ist einerseits eine hervorragende Verformbarkeit gewährleistet. Andererseits gewährt die optimale Abstimmung der Eigenschaften der einzelnen Schichten der Beschichtung einen ebenso guten und lange wirkenden Korrosionsschutz.

Im Ergebnis ermöglicht es die Erfindung, ein Stahlband mit einer Zinkbeschichtung zu versehen, die einen gegenüber dem Stand der Technik verbesserten Korrosionsschutz gewährleistet. Dazu werden ein bestimmter Aluminium-Gehalt der Zinkbäder ausgewählt, bestimmte Verweilzeiten des Stahlbandes in den Tauchbädern eingehalten, bestimmte Temperaturen der Tauchbäder eingestellt und eine Abkühlung zwischen den Tauchbädern durchgeführt.

Die Glühtemperatur beträgt vorzugsweise 400 °C bis 800 °C. Indem das Durchlaufglühen unter.Schutzgas stattfindet, wird eine Oxidation der Bandoberfläche sicher vermieden.

Praktische Erprobungen des erfindungsgemäßen Verfahrens haben gezeigt, daß sich gute Verarbeitungsergebnisse einstellen, wenn die Tauchzeiten 1 bis 20 Sekunden dauern. Dabei kann die Qualität der Beschichtung, unter Berücksichtigung des jeweils verwendeten Schmelzenbades und der jeweils eingestellten Badtemperatur, dadurch weiter gesteigert werden, daß die Tauchzeiten innerhalb eines Bereiches von 3 bis 10 Sekunden liegen.

Zusätzliche Verbesserungen des Beschichtungsergebnisses lassen sich durch eine Optimierung der Schmelzbadzusammensetzung erreichen. In der Praxis hat sich gezeigt, daß es günstig ist, wenn die erste Zinkschmelze 0,005 - 0,25 % Gew. Aluminium enthält, wobei sich besonders gute Ergebnisse einstellen, wenn die erste Zinkschmelze 0,01 - 0,12 Gew.-% Aluminium beinhaltet. Bei Verwendung einer derart zusammengesetzten Schmelze in der ersten Stufe der Schmelztauchbeschichtung bildet sich auf dem Stahlband als Grundschicht eine Zn-Fe-Legierung, die besonders fest auf der Oberfläche des Stahlbands haftet und eine Deckschicht aus Zink bildet. In der zweiten Stufe der Schmelztauchverzinkung wird dann die in der ersten Stufe gebildete Grundschicht umgewandelt in eine Zn-Al-Fe-Legierung und eine Zn-Al-Deckschicht gebildet.

Eine qualitativ hochwertige Beschichtung kann dabei dadurch gebildet werden, wenn die im zweiten Beschichtungsgang aufgebrachte zweite Zinkschmelze 3 Gew.-% - 15 Gew.-%, insbesondere 4 Gew.-% bis 6 Gew.-%, Aluminium enthält. Eine derart zusammengesetzte, auf die Grundschicht aufgetragene Schmelze bildet eine ZnAl-Deckschicht, die sich durch eine hohe Duktilität und große Korrosionsbeständigkeit auszeichnet.

Die Temperatur, mit welcher die Aluminium in der angegebenen Menge enthaltende Zinkschmelze in der ersten Stufe der Tauchschmelzverzinkung aufgebracht wird, sollte 440 - 490 °C betragen. Bei Einhaltung dieses Temperaturbereichs läßt sich problemlos eine Grundschicht erzeugen, welche die gewünschte Schichtdicke besitzt.

Optimale Beschichtungsergebnisse lassen sich während der zweiten Stufe der Beschichtung dann erreichen, wenn die Temperatur im zweiten Tauchbad 420 °C - 480 °C beträgt.

Im Anschluß an die Zinkbeschichtung kann das beschichtete Stahlband mit einer organischen Schutzschicht versehen werden, welche einen zusätzlichen Schutz der Oberfläche gegen Beschädigung während der Lagerung oder des Transports bildet.

Zweckmäßig in Bezug auf die Erzeugung besonders hochwertiger Stahlbänder ist es darüber hinaus, wenn das beschichtete Stahlband nach dem zweiten Tauchbad abgekühlt wird. Praktische Versuche haben in diesem Zusammenhang gezeigt, daß sich besonders gute Ergebnisse einstellen, wenn bei der Abkühlung nach dem zweiten Tauchbad die Abkühlgeschwindigkeit mindestens 4 °C / s beträgt.

Das erfindungsgemäße Verfahren ermöglicht es, kalt- oder warmgewalzte Stahlbänder aus beruhigtem, niedrig gekohltem Stahl, aus mikrolegiertem ULC-Stahl oder hoch- und höchstfestem Stahl mit einer mindestens zweischichtigen Beschichtung zu versehen, die aus einer auf der Oberfläche des Stahlbandes aus einer Zinkschmelze mit einer ersten Zusammensetzung erzeugten Grundschicht und mindestens einer auf der Grundschicht aufgetragenen Deckschicht gebildet ist, welche aus einer Zinkschmelze erzeugt ist, die sich von der Zusammensetzung der ersten Zinkschmelze unterscheidet. Dabei sind die Zusammensetzungen der Zinkschmelzen zweckmäßigerweise so gewählt, daß die Grundschicht aus ZnAlFe und die Deckschicht aus ZnAl gebildet ist, wobei, wie schon voranstehend erwähnt, die zur Erzeugung der Grundschicht verwendeten Schmelze neben Zink 0,005 - 0,25 % Aluminium und die zur Erzeugung der Schmelze der Deckschicht neben Zink 3,5 - 15 % Aluminium enthalten kann.

Erfindungsgemäß erzeugtes Stahlband ist mit einer mindestens aus einer Grundschicht und einer darauf aufgetragenen Deckschicht gebildeten Beschichtung beschichtet. Der erfindungsgemäß erzeugte, mindestens zweischichtige Aufbau ermöglicht es, die einzelnen Schichten der Beschichtung so aufeinander abzustimmen, daß sich ihre Eigenschaften in der voranstehend im Zusammenhang mit dem erfindungsgemäßen Verfahren erläuterten Weise optimal ergänzen. Dabei ergeben sich besonders günstige Eigenschaftskombinationen, wenn die Grundschicht im wesentlichen aus Zn-Al-Fe und die Deckschicht im wesentlichen aus Zn-Al besteht.

Vorzugsweise sind die Aluminium-Gehalte der einzelnen Schichten der auf das Stahlband aufgebrachten Beschichtung so gewählt, daß der Aluminium-Gehalt der Beschichtung mindestens 4 Gew.-% beträgt. Dies läßt sich beispielsweise basierend auf einer Grundschicht erreichen, die einen Aluminium-Gehalt von mindestens 5 Gew.-% besitzt. Grundsätzlich ist es im Hinblick auf die Verformbarkeit des beschichteten Stahlbandes darüber hinaus zweckmäßig, wenn der Aluminium-Gehalt der Deckschicht niedriger ist als der Aluminium-Gehalt der Grundschicht. So besitzen Stahlbänder, die mit einer erfindungsgemäß erzeugten Beschichtung beschichtet sind und im Bereich der Deckschicht einen Aluminium-Gehalt von mindestens 3 Gew.-% aufweisen, besonders gute Verformungseigenschaften bei gleichzeitig hoher Korrosionsbeständigkeit.

Der Anteil der Dicke der Grundschicht an der aus der Summe der Dicken von Grund- und Deckschicht gebildeten Gesamtdicke sollte vorzugsweise mindestens 10 % betragen. Auf diese Weise ist einerseits ein sicheres Anhaften der Grundschicht auf dem Stahlband gewährleistet. Andererseits ist auf diese Weise die Voraussetzung für die Erzeugung einer ausreichend dicken und gut haftenden Deckschicht geschaffen. Erfindungsgemäß erzeugte Stahlbänder weisen dabei Gesamtdicken der Beschichtung von mindestens 20 µm auf.

Nachfolgend wird die Erfindung anhand einer ein Ausführungsbeispiel darstellenden Zeichnung näher erläutert. Es zeigen in schematischer Darstellung:

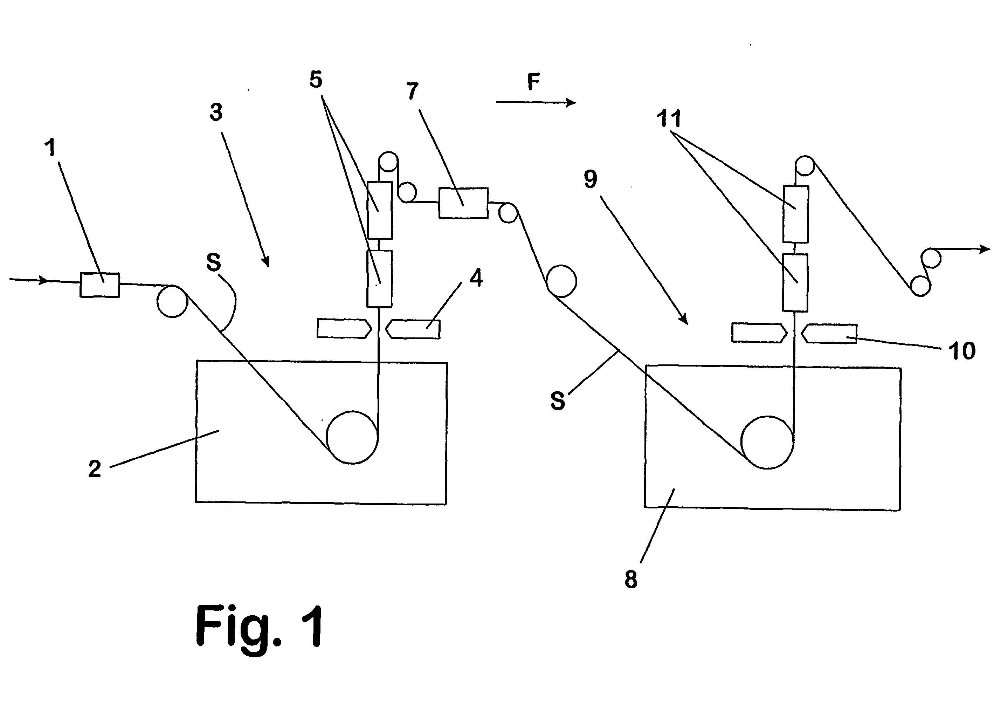

- Fig. 1: einen Ausschnitt einer Feuerverzinkungsanlage von Stahlbändern;
- Fig. 2: einen Schnitt durch ein mit einer zweischichtigen Zinkbeschichtung versehenes Stahlband.

Ein Stahlband S, bei dem es sich beispielsweise um ein warmgewalztes Feinblech aus einem Al-beruhigtem, niedrig Kohlenstoff haltigem Stahl handelt, wird von einem nicht gezeigten Abwickler abgewickelt und in einer ebenso nicht gezeigten Vorbehandlungseinrichtung in üblicher Weise entfettet und in verdünnter Säure gebeizt. Anschließend wird das Stahlband S in einem Durchlaufofen 1 unter Schutzgas bei einer Temperatur von beispielsweise 600 °C durchlaufgeglüht.

Das derart vorbereitete Stahlband S wird mit einer Geschwindigkeit zwischen 10 und 50 m/min in Förderrichtung F in das Schmelzenbad 2 einer ersten Schmelztaucheinrichtung 3 gefördert.

Das Schmelzenbad 2 der Schmelztaucheinrichtung 3 ist durch eine Zinkschmelze gebildet, welche einen Aluminiumgehalt von beispielsweise 0,05 Gew.-% aufweist. Die Temperatur des Schmelzenbades 2 beträgt 460 °C. Die für einen Durchlauf des Schmelzenbades 2 benötigte Zeit beträgt beispielsweise ca. 10 s.

Im Anschluß an den Austritt aus dem Schmelzenbad 2 durchläuft das Stahlband S eine Düsenabstreifeinrichtung 4. In der Düsenabstreifeinrichtung 4 wird die Dicke d1 der durch die Zinkschmelze des Schmelzenbades 2 gebildeten Grundschicht G eingestellt, indem die überschüssige Menge der auf der Oberfläche O des Stahlbandes S haftenden, noch flüssigen Schmelze über Schlitzdüsen von dem Stahlband S geblasen wird.

Anschließend durchläuft das Stahlband S eine Kühleinrichtung 5, in welcher es auf eine Temperatur von 250 °C abgekühlt wird. Im Zuge dieser Abkühlung erstarrt die auf der Oberfläche O des Stahlbandes S haftende Schmelze.

Auf die Kühleinrichtung 5 folgt eine Flux-Einrichtung 7, in welcher das Stahlband S erforderlichenfalls einer weiteren Benetzungsbehandlung mit anschließender Trocknung unterzogen wird.

Anschließend läuft das abgekühlte und erforderlichenfalls nochmals einer Benetzungsbehandlung unterzogene Stahlband S in das Schmelzenbad 8 einer zweiten Schmelztaucheinrichtung 9 ein.

Das Schmelzenbad 8 der Schmelztaucheinrichtung 9 ist durch eine Zinkschmelze gebildet, welche neben Zink einen Aluminiumgehalt von beispielsweise 4,5 Gew.-% aufweist. Die Temperatur des Schmelzenbades 8 beträgt ebenfalls 460 °C. Die für einen Durchlauf des Schmelzenbades 8 benötigte Tauchzeit beträgt beispielsweise ca. 10 s.

Im Anschluß an den Austritt aus dem Schmelzenbad 8 durchläuft das Stahlband S eine Blaseinrichtung 10. In der Blaseinrichtung 10 wird die Dicke d2 der durch die Zinkschmelze des Schmelzenbades 8 auf der Grundschicht G gebildeten Deckschicht D eingestellt, indem die überschüssige Menge der auf der Oberfläche O' der Grundschicht G haftenden, noch flüssigen Schmelze über Schlitzdüsen von der Oberfläche O' der Grundschicht G geblasen wird.

Auf die Blaseinrichtung 10 folgt in Förderrichtung F eine weitere Kühleinrichtung 11, in welcher das beschichtete Stahlband S mit einer Abkühlgeschwindigkeit von 40 K/s auf Raumtemperatur abgekühlt wird.

In einer nicht dargestellten Beschichtungseinrichtung wird das Stahlband S dann noch mit einer zusätzlichen Schutzschicht versehen, um einen temporären Oberflächenschutz herzustellen.

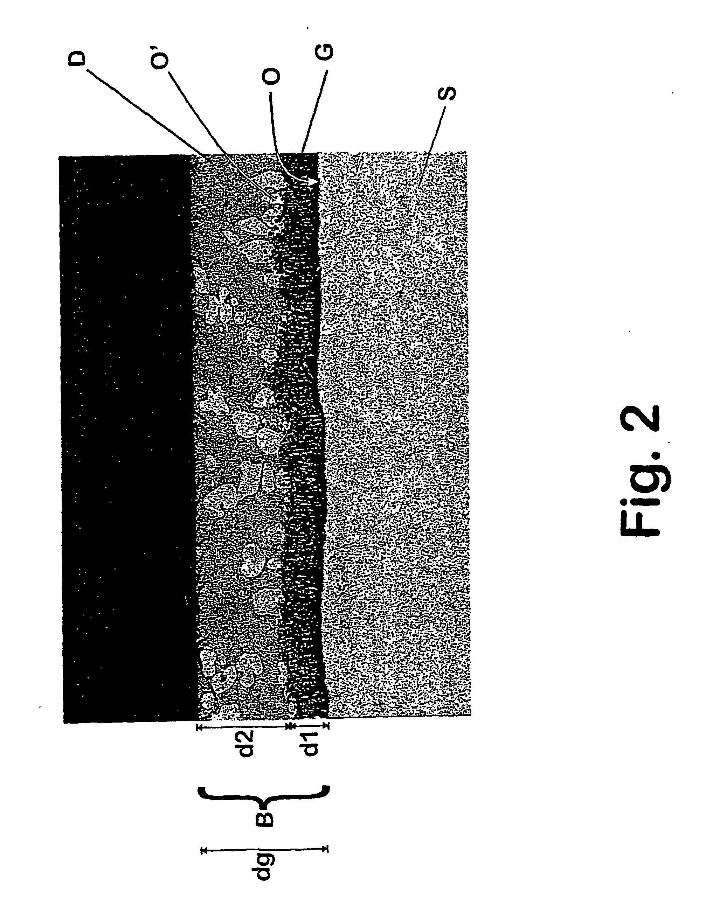

Aus Fig. 2 ist ersichtlich, daß im Ergebnis ein Stahlband S erzeugt wird, welches mit einer fest auf ihm haftenden Beschichtung B versehen ist. Diese Beschichtung B ist einerseits durch eine Grundschicht G gebildet, welche wegen des Einflusses des im Stahl des Stahlbandes S enthaltenen Eisens neben Zn und Al auch Fe enthält. Die Dicke d1 der Grundschicht G beträgt etwa 25 % der aus der Dicke d1 der Grundschicht G und der Dicke d2 der Deckschicht D gebildeten Gesamtdicke dg der Beschichtung B.

Aus Fig. 2 ist deutlich zu erkennen, wie die Grundschicht G auf dem Stahlband S "aufgewachsen" ist und wie die Schmelze der Deckschicht D während des Aufenthalts im zweiten Schmelzbad 8 in die Grundschicht G diffundiert ist, so daß eine innige Verbindung zwischen der Grundschicht G und der Deckschicht D entstanden ist.

In den nachfolgenden Tabellen I - III sind jeweils die Ergebnisse von Versuchen dargestellt, die mit Hilfe eines Schmelztauchsimulators durchgeführt worden sind.

Die Versuche, deren Ergebnisse in Tabelle I angegeben sind, wurden mittels eines Rhesca-Schmelztauchsimulators im Labormaßstab durchgeführt. Dabei wurden die Aluminiumgehalte im ersten Zinkbad auf 0,1 bis 0,2 Gew.-% eingestellt. Derartige Aluminiumgehalte werden in Bandverzinkungsanlagen bei der einstufigen Einmalverzinkung standardmäßig gefahren (s. Beispiele 1 und 2 der Tabelle I) und sind für eine gute Zinkhaftung notwendig.

Alternativ werden bei der Einmalverzinkung in Bandverzinkungsanlagen auch Aluminiumgehalte von bis zu 5 % eingestellt. Die dazu verwendeten Schmelzenzusammensetzungen sind unter dem Produktnamen "Galfan" marktbekannt. Beispiel 4 der Tabelle I zeigt das Ergebnis, welches sich bei Verwendung einer 5 % Aluminium enthaltenden Zinkschmelze in einer Einmalverzinkung einstellen.

In Bandverzinkungsanlagen mit Fluxvorbehandlung werden jedoch keine Einmalverzinkungen in Aluminiumbädern mit bis zu 5 % durchgeführt, da es dabei zu Beschichtungsfehlern kommt. Beispiel 3 der Tabelle I bestätigt diese Feststellung.

Neben den Aluminiumgehalten sind bei den Beispielen 5 - 7 der Tabelle I, die gemäß der Erfindung zweistufig und mit einer vorangehenden Glühbehandlung verzinkt worden sind, die Tauchzeiten im zweiten Schmelzbad zwischen 10 und 20 Sekunden variiert worden. Es wurde zum Teil "mit" und zum Teil "ohne" Zwischenflux gearbeitet. Die bei der abschließenden Abkühlung erreichten Abkühlgeschwindigkeiten wurden zwischen 4,5 °C/s und 40 °C/s verändert. Die Beispiele 5 bis 7 der Tabelle I zeigen, daß unter diesen Bedingungen Stahlbänder mit Beschichtungen erzeugt werden können, die besonders gute Haftungseigenschaften, ein hohes Umformvermögen und gute Korrossionsbeständigkeit besitzen.

Auch die in Tabelle II angegebenen Ergebnisse wurden in Versuchen ermittelt, die mittels des Rhesca-Schmelztauchsimulators im Labormaßstab durchgeführt worden sind. In der in Tabelle II dargestellten Versuchsserie sind die Aluminiumgehalte im ersten Zinkbad auf 0,08 bzw. 0,05 Gew.-% abgesenkt worden. Dies führte bei den einstufig durchgeführten Verzinkungen sowohl bei einer Fluxvorbehandlung als auch bei einer Glühvorbehandlung zu schlechten Haftergebnissen, wie dies beispielsweise an den Ergebnissen der Beispiele 8 und 9 abzulesen ist.

Bei den in erfindungsgemäßer Weise zweistufig durchgeführten Verzinkungen wurden die Tauchzeiten der zweiten Verzinkung konstant auf 10 Sekunden festgesetzt. Varriert wurden dagegen nochmals die Zwischenfluxbehandlung ("mit" und "ohne") sowie die Abkühlgeschwindigkeiten bei der abschließenden Kühlung, welche zwischen 4,5 °C/s und 40 °C/s eingestellt worden sind. Die in Tabelle II angegebenen Beispiele 10 bis 14 zeigen, daß die nach dem erfindungsgemäßen Verfahren erzeugten Proben Beschichtungen mit besonders guter Haftung und zum Teil überragenden Standzeiten im Korrosionstest aufweisen.

Die Versuche, deren Ergebnisse in der Tabelle III dargestellt sind, wurden ebenfalls in Versuchen gewonnen, die im Rhesca-Schmelztauchsimulator durchgeführt worden sind. In dieser Versuchsserie sind die Aluminiumgehalte im ersten Zinkbad gegenüber den zuvor erläuterten Versuchsserien noch weiter abgesenkt worden. Sie lagen bei 0,01 Gew.-%. Dennoch zeichnen sich alle Beispiele der Tabelle III durch eine gute Haftung und einen sehr hohen Korrosionswiderstand aus.

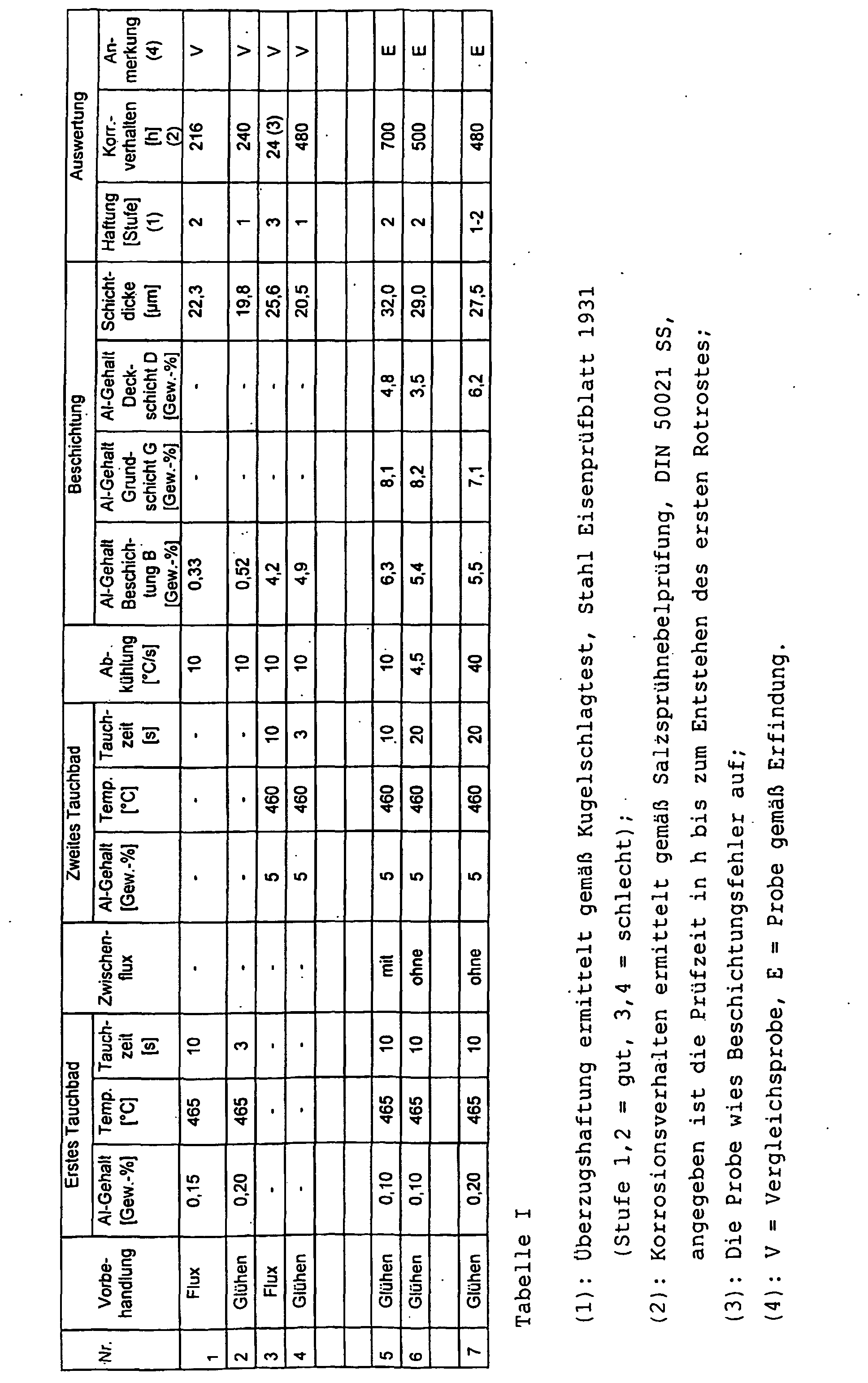

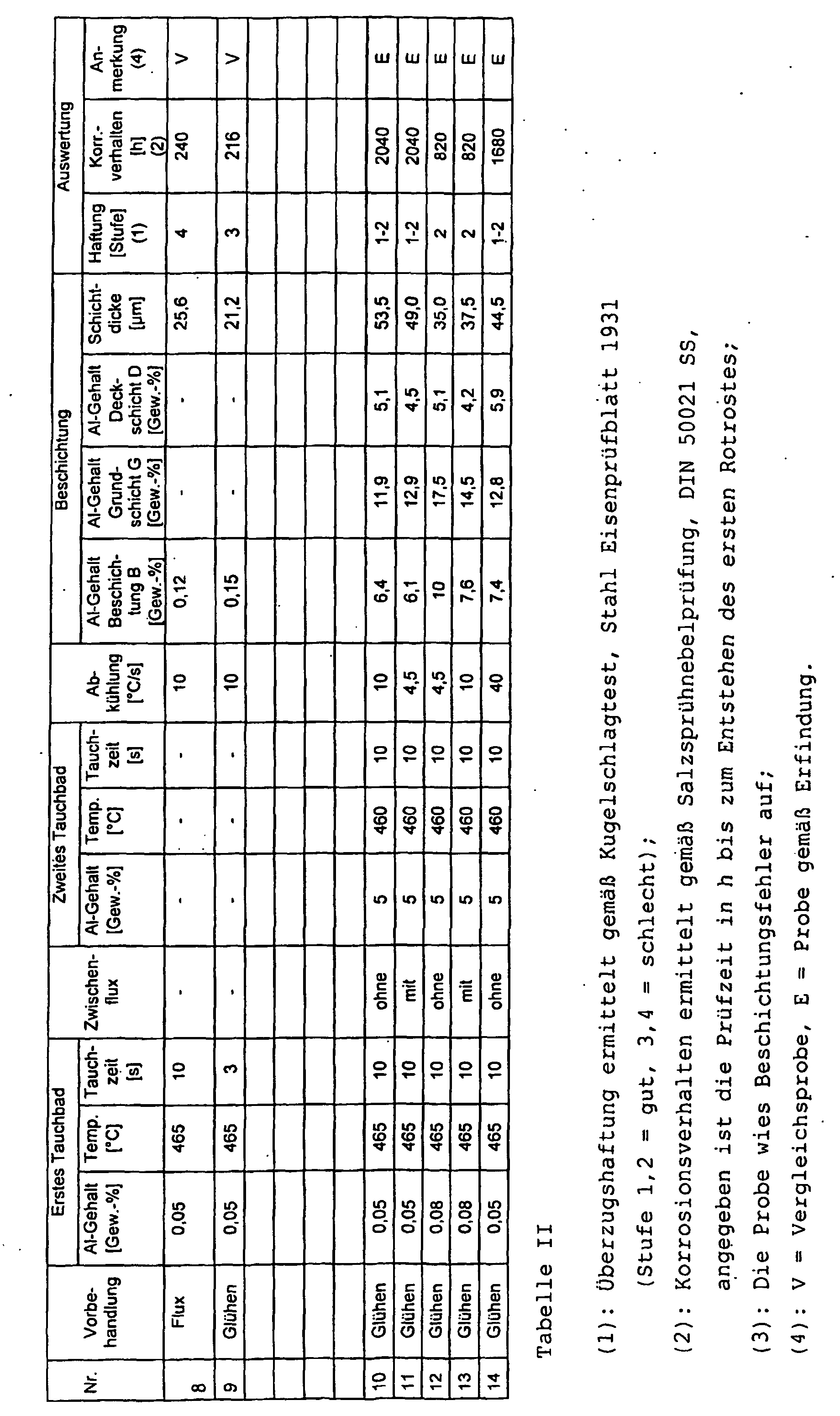

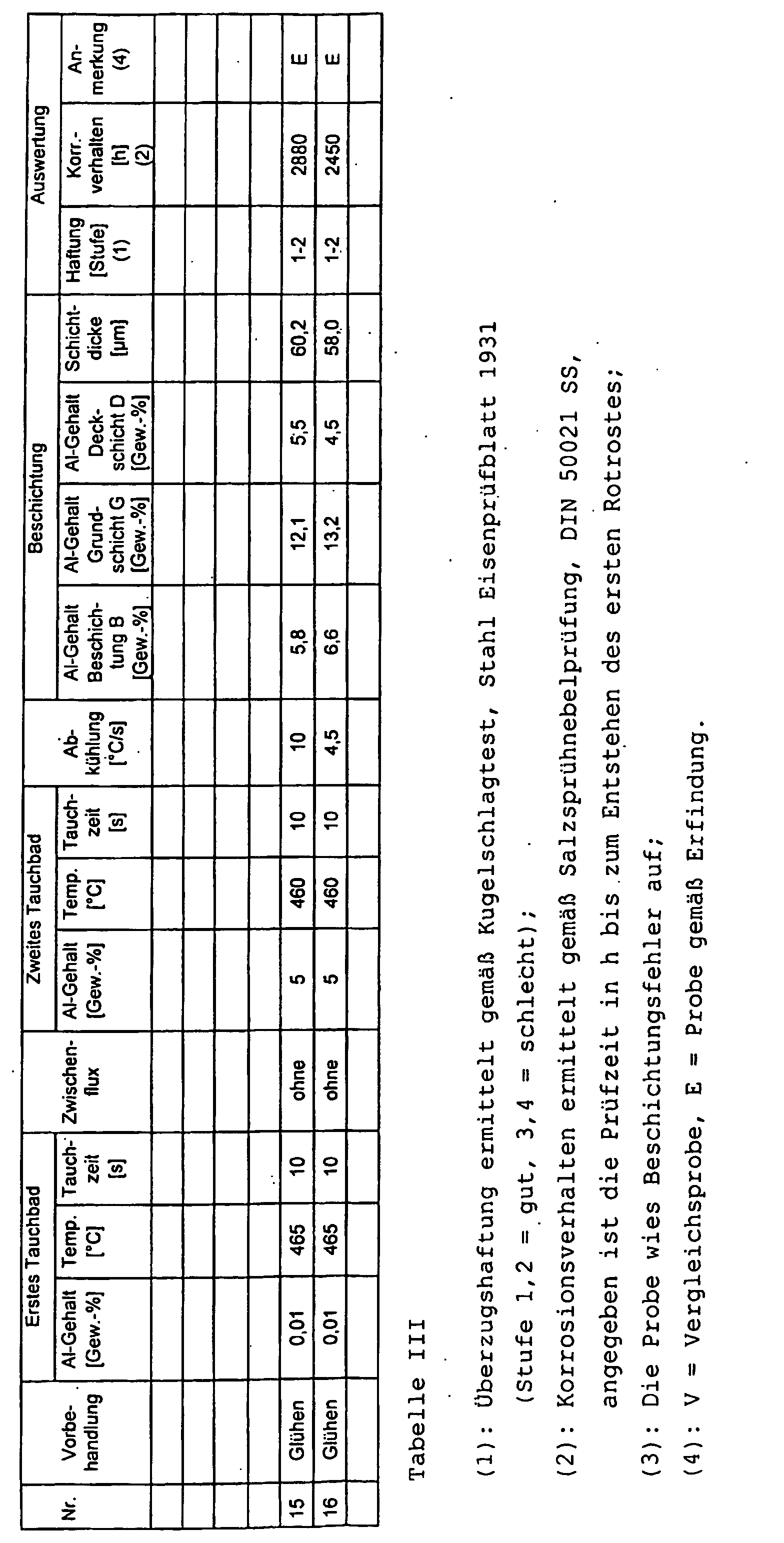

### BEZUGSZEICHENLISTE

- 1: Flux-Einrichtung
- 2: Schmelzenbad
- 3: Schmelztaucheinrichtung
- 4: Düsenabstreifeinrichtung
- 5: Kühleinrichtung
- 7: Flux-Einrichtung
- 8: Schmelzenbad
- 9: Schmelztaucheinrichtung
- 10: Blaseinrichtung
- 11: Kühleinrichtung

- B: Beschichtung
- D: Deckschicht
- G: Grundschicht
- d1: Dicke der Grundschicht G
- d2: Dicke der Deckschicht D
- dg: Gesamtdicke der Beschichtung B
- O: Oberfläche des Stahlbandes S
- O': Oberfläche der Grundschicht G
- S: Stahlband

## Patentansprüche

1. Verfahren zum Herstellen eines mit einer Zinkbeschichtung versehenen Stahlbandes (S), bei dem das Stahlband (S) kontinuierlich aufeinanderfolgend die folgenden Arbeitsschritte durchläuft und dabei einem mindestens zweistufig erfolgenden Schmelztauchverzinken unterzogen wird:
- Durchlaufglühen des Stahlbands (S) vor dem Eintritt in das erste Tauchbad,
Beschichten des Stahlbands (S) in der ersten Stufe der Schmelztauchverzinkung mit einer Grundschicht (G), indem das Stahlband für eine erste Tauchzeit durch ein erstes Tauchbad (2) aus einer einen niedrigen Aluminium-Gehalt aufweisenden Zinkschmelze geleitet wird,
- Abkühlen des mit der Grundschicht (G) beschichteten Stahlbands (S) und
- Aufbringen einer Deckschicht (D) auf das mit der Grundschicht (G) beschichtete Stahlband (S), indem in der zweiten Stufe der Schmelztauchbeschichtung das mit der Grundschicht (G) beschichtete Stahlband (S) für eine zweite Tauchzeit durch ein zweites Tauchbad (8) geleitet wird, welches aus einer einen höheren Aluminium-Gehalt als die erste Zinkschmelze besitzenden zweiten Zinkschmelze gebildet ist.

2. Verfahren nach Anspruch 1, **dadurch gekennzeichnet, daß** die Tauchzeiten 1 bis 20 Sekunden dauern.

3. Verfahren nach Anspruch 2, **dadurch gekennzeichnet, daß** die Tauchzeiten im Bereich von 3 bis 10 Sekunden liegen.

4. Verfahren nach einem der voranstehenden Ansprüche, **dadurch gekennzeichnet, daß** die erste Zinkschmelze 0,005 Gew.-% - 0,25 Gew.-%, bevorzugt 0,01 bis 0,12 Gew.-%, Aluminium enthält.

5. Verfahren nach einem der voranstehenden Ansprüche, **dadurch gekennzeichnet, daß** die zweite Zinkschmelze 3 Gew.-% - 15 Gew.-% Aluminium enthält.

6. Verfahren nach Anspruch 5, **dadurch gekennzeichnet, daß** die zweite Zinkschmelze 4 Gew.-% - 6 Gew.-% Aluminium enthält.

7. Verfahren nach einem der voranstehenden Ansprüche, **dadurch gekennzeichnet, daß** die Temperatur der Zinkschmelze im ersten Tauchband (2) 440 °C - 490 °C beträgt.

8. Verfahren nach einem der voranstehenden Ansprüche, **dadurch gekennzeichnet, daß** die Temperatur im zweiten Tauchbad (8) 420 °C - 480 °C beträgt.

9. Verfahren nach einem der voranstehenden Ansprüche, **dadurch gekennzeichnet, daß** die Glühtemperatur zwischen 400 und 800 °C beträgt.

10. Verfahren nach einem der voranstehenden Ansprüche, **dadurch gekennzeichnet, daß** das Durchlaufglühen unter Schutzgas erfolgt.

11. Verfahren nach einem der voranstehenden Ansprüche, **dadurch gekennzeichnet, daß** nach der Schmelztauchverzinkung auf die Deckschicht (D) eine Schutzschicht aufgetragen wird, durch welche die Oberfläche der Deckschicht (D) geschützt wird.

12. Verfahren nach einem der voranstehenden Ansprüche, **dadurch gekennzeichnet, daß** das beschichtete Stahlband (S) nach dem zweiten Tauchbad (8) abgekühlt wird.

13. Verfahren nach Anspruch 12, **dadurch gekennzeichnet, daß** die Abkühlgeschwindigkeit mindestens 4 °C / s beträgt.

## Claims

1. A method for manufacturing a steel strip (S) provided with a zinc coating, where the steel strip (S) passes continuously through the following working stages and is thereby subjected to an at least two-stage hot-dip zinc plating:
- Continuous annealing of the steel strip (S) before entry into the first dipping bath, Coating of the steel strip (S) in the first stage of the hot-dip zinc plating with a base layer (G), wherein the steel strip is passed for a first dipping time through a first dipping bath (2) comprising a zinc melt having a low aluminium content,
- Cooling of the steel strip (S) coated with the base layer (G),
- Application of a cover layer (D) to the steel strip (S) coated with the base layer (G) wherein in the second stage of the hot-dip zinc plating the steel strip (S) coated with the base layer (G) is passed for a second dipping time through a second dipping bath (8) which is formed from a second zinc melt having a higher aluminium content than the first zinc melt.

2. The method according to Claim 1, **characterised in that** the dipping times are 1 to 20 seconds.

3. The method according to Claim 2, **characterised in that** the dipping times lie in the range of 3 to 10 seconds.

4. The method according to one of the preceding claims, **characterised in that** the first zinc melt contains 0.005 wt. % - 0.25 wt. %, preferably 0.01 to 0.12 wt. % aluminium.

5. The method according to one of the preceding claims, **characterised in that** the second zinc melt contains 3 wt. % - 15 wt. % aluminium.

6. The method according to Claim 5, **characterised in that** the second zinc melt contains 4 wt. % - 6 wt. % aluminium.

7. The method according to any one of the preceding claims, **characterised in that** the temperature of the zinc melt in the first dipping bath (2) is 440°C - 490°C.

8. The method according to any one of the preceding claims, **characterised in that** the temperature in the second dipping bath (8) is 420°C - 480°C.

9. The method according to any one of the preceding claims, **characterised in that** the annealing temperature is between 400 and 800°C.

10. The method according to any one of the preceding claims, **characterised in that** the continuous annealing takes place under protective gas.

11. The method according to any one of the preceding claims, **characterised in that** after the hot-dip zinc plating a protective layer is applied to the cover layer (D) through which the surface of the cover layer (D) is protected.

12. The method according to any one of the preceding claims, **characterised in that** the coated steel strip (S) is cooled after the second dipping bath (8).

13. The method according to Claim 12, **characterised in that** the cooling rate is at least 4°C/s.

## Revendications

1. Procédé de préparation d'une bande d'acier (S) munie d'un revêtement de zinc, dans lequel la bande d'acier (S) passe en continu, successivement, les étapes suivantes et est ainsi soumise à un zingage à chaud en au moins deux étapes :
- recuit au passage de la bande d'acier (S) avant l'entrée dans le premier bain d'immersion,
revêtement de la bande d'acier (S) dans une première étape du zingage à chaud, avec une couche de base (G), dans lequel la bande d'acier est passée pendant une première durée d'immersion, dans un premier bain d'immersion (2) d'une masse fondue de zinc présentant une teneur faible en aluminium,
- refroidissement de la bande d'acier (S) revêtue de la couche de base (G), et
- application d'une couche de couverture (D) sur la bande d'acier (S) revêtue de la couche de base (G), dans lequel dans la deuxième étape du revêtement par immersion, la bande d'acier (S) revêtue de la couche de base (G) est passée pendant une deuxième durée d'immersion, dans un deuxième bain d'immersion (8), qui est formé d'une deuxième masse fondue de zinc possédant une teneur en aluminium plus élevée que la première masse fondue de zinc.

2. Procédé selon la revendication 1, **caractérisé en ce que** les durées d'immersion durent 1 à 20 secondes.

3. Procédé selon la revendication 2, **caractérisé en ce que** les durées d'immersion se situent dans l'intervalle allant de 3 à 10 secondes.

4. Procédé selon l'une des revendications précédentes, **caractérisé en ce que** la première masse fondue de zinc contient 0,005% en poids - 0,25% en poids, de préférence 0,01 - 0,12% en poids d'aluminium.

5. Procédé selon l'une des revendications précédentes, **caractérisé en ce que** la deuxième masse fondue de zinc contient 3% en poids - 15% en poids d'aluminium.

6. Procédé selon la revendication 5, **caractérisé en ce que** la deuxième masse fondue de zinc contient 4% en poids - 6% en poids d'aluminium.

7. Procédé selon l'une des revendications précédentes, **caractérisé en ce que** la température de la masse fondue de zinc dans le premier bain d'immersion (2) se situe dans l'intervalle allant de 440°C à 490°C.

8. Procédé selon l'une des revendications précédentes, **caractérisé en ce que** la température de la masse fondue de zinc dans le deuxième bain d'immersion (8) se situe dans l'intervalle allant de 420°C à 480°C.

9. Procédé selon l'une des revendications précédentes, **caractérisé en ce que** la température de recuit se situe dans l'intervalle allant de 400 à 800°C.

10. Procédé selon l'une des revendications précédentes, **caractérisé en ce que** le recuit de passage est réalisé sous gaz protecteur.

11. Procédé selon l'une des revendications précédentes, **caractérisé en ce qu'**après le zingage à chaud, on applique sur la couche de couverture (D), une couche de protection, par laquelle la surface de la couche de couverture (D) est protégée.

12. Procédé selon l'une des revendications précédentes, **caractérisé en ce que** la bande d'acier (S) revêtue est refroidie après le deuxième bain d'immersion (8).

13. Procédé selon la revendication 12, **caractérisé en ce que** la vitesse de refroidissement s'élève à au moins 4°C/seconde.
